# EUROPÄISCHE PATENTSCHRIFT

(11) **EP 1 226 107 B1**
(45) Veröffentlichungstag und Bekanntmachung des Hinweises auf die Patenterteilung: **05.10.2005**
(21) Anmeldenummer: 01913893.2
(22) Anmeldetag: 19.03.2001
(51) Int. Cl.: C07C 211/51, C07C 215/50, C07C 217/58, C07D 295/12, C07C 215/14, C07C 233/43

(54) **N-BENZYL-P-PHENYLENDIAMIN-DERIVATE ENTHALTENDE FÄRBEMITTEL FÜR KERATINFASERN SOWIE NEUE N-BENZYL-P-PHENYLENDIAMIN-DERIVATE**
N-BENZYL-P-PHENYLENEDIAMINE-DERIVATIVES CONTAINING COLOURING AGENTS FOR KERATIN FIBRES AND NOVEL N-BENZYL-P-PHENYLENEDIAMINE-DERIVATIVES
COLORANTS POUR FIBRES DE KERATINE CONTENANT DES DERIVES N-BENZYL-P-PHENYLENDIAMINE, ET NOUVEAUX DERIVES N-BENZYL-P-PHENYLENDIAMINE

(30) Priorität: 31.08.2000 DE 10042787
(43) Veröffentlichungstag der Anmeldung: 31.07.2002
(60) Teilanmeldung: 04022498.2
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/003121
(87) Internationale Veröffentlichungsnummer: WO 2002/018318

(56) Entgegenhaltungen:
- EP-A- 0 024 660
- DE-A- 3 432 214
- US-A- 5 180 399
- CHEMICAL ABSTRACTS, vol. 128, no. 23, 8. Juni 1998 (1998-06-08) Columbus, Ohio, US; abstract no. 282671, CHEREZOVA, E. N. ET AL: "Reaction of dimethyl(3,5-di-tert-butyl-4-hydroxybenzyl )amine with substituted arylamines" XP002169308 & RUSS. J. GEN. CHEM. (1997), 67(6), 932-935 , 1997,
- DATABASE CROSSFIRE [Online] Beilsteininformationssystme ; XP002169310 & PAAL; POLLER: J. PRAKT. CHEM. <2>54, 1896,272,
- CHEMICAL ABSTRACTS, vol. 65, no. 5, 29. August 1966 (1966-08-29) Columbus, Ohio, US; abstract no. 7001f, G. N. WALKER ET AL.: "Synthesis of varied heterocyclic and substituted aryl alkyl secondary amines, related Schiff bases, and amides" XP002169309 & J. MED. CHEM., Bd. 9, Nr. 4, 1966, Seiten 624-30,

## Beschreibung

Die vorliegende Erfindung betrifft Mittel zur oxidativen Färbung von Keratinfasem, insbesondere menschlichen Haaren, auf der Basis einer Entwickler-substanz/Kupplersubstanz-Kombination, welche als Entwicklersubstanz N-Benzyl-p-phenylendiamin-Derivate enthalten, sowie neue N-Benzyl-p-phenylendiamin-Derivate.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in-der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Aus der DE-OS 34 32 214 sind bereits Mittel zum Färben von Haaren mit einem Gehalt an bestimmten N-Benzyl-p-phenylendiamin, beispielsweise N-Benzyl-p-phenylendiamin, N4-Benzyl-1,4-diamino-2-methylbenzol und 2-(((4-aminophenyl)amino)methyl)-4,6-dichlor-phenol, bekannt. Diese Verbindungen erfüllen jedoch die an Farbstoffe für Oxidationsfärbemittel gestellten Anforderungen nicht in jeder Hinsicht. Es bestand daher weiterhin ein Bedarf nach geeigneten neuen Farbstoffen.

Es wurde nun gefunden, dass bei Verwendung von N-Benzyl-p-phenylendiamin-Derivaten der allgemeinen Formel (I) intensive braune, blaue und rote Farbnuancen erhalten werden.

Gegenstand der vorliegende Erfindung ist daher ein Mittel zur oxidativen Färbung von Keratinfasem, wie zum Beispiel Wolle, Pelzen, Federn oder Haaren, insbesondere menschlichen Haaren, auf der Basis einer Entwicklersubstanz-Kupplersubstanz-Kombination, das als Entwicklersubstanz mindestens ein N-Benzyl-p-phenylendiamin-Derivat der Formel (I) enthält, 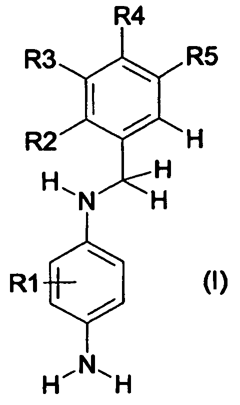 worin
- **R1**: gleich Wasserstoff, einer (C₁-C₄)-Alkylgruppe oder einer Hydroxy-(C₁-C₄)-alkylgruppe ist;
- **R2**: gleich Wasserstoff, einem Halogenatom (F, Cl, Br, J), einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy(C₁-C₄)-alkyl)-aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder einer Morpholinogruppe ist;
- **R3**: Wasserstoff, ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
- R4: ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt,
oder R3 und R4 gemeinsam eine -O-CH2-O-Brücke bilden;
- R5: gleich Wasserstoff, einer Hydroxygruppe oder einer (C₁-C₆)-Alkylgruppe ist.

Als Verbindungen der Formel (I) können beispielweise genannt werden: N-((4-Hydroxy-phenyl)methyl)-1,4-diaminobenzol, N-((4-Hydroxy-3,5-dimethyl-phenyl)methyl)-1,4-diaminobenzol, N-((4-(1-Hydroxyethoxy)-phenyl)methyl)-1,4-diaminobenzol, N-((4-Methoxyphenyl)methyl)-1,4-diaminobenzol, N-((4-(2-Hydroxyethylamino)-phenyl)methyl)-1,4-diaminobenzol, N-((4-(Bis-(2-hydroxyethyl)amino)-phenyl)methyl)-1,4-diaminobenzol, N-((4-Dimethylamino-phenyl)methyl)-1,4-diaminobenzol, N-((4-Pyrrolidino-phenyl)methyl)-1,4-diaminobenzol, N-Benzo[1,3]dioxol-5-ylmethyl-1,4-diaminobenzol, N-{4-[(4-Amino-phenylamino)-methyl]-phenyl}-acetamid, N-((2,4-Diaminophenyl)methyl)-1,4-diaminobenzol, N-((2,4-Dihydroxy-phenyl)methyl)-1,4-diaminobenzol, N¹-((4-Hydroxyphenyl)methyl)-2-(2-hydroxyethyl)-1,4-diaminobenzol, N¹-((4-Hydroxyphenyl)methyl)-2-methyl-1,4-diaminobenzol, N⁴-((4-Hydroxyphenyl)methyl)-2-(2-hydroxy-ethyl)-1,4-diaminobenzol und N⁴-((4-Hydroxyphenyl)methyl)-2-methyl-1,4-diaminobenzol.

Besonders bevorzugt sind die folgenden N-Benzyl-p-phenylendiamin-Derivate der Formel (I): N-((4-Hydroxyphenyl)-methyl)-1,4-diaminobenzol; N-((4-Hydroxy-3,5-dimethyl-phenyl)methyl)-1,4-diaminobenzol; N-((4-(2-Hydroxyethoxy)-phenyl)methyl)-1,4-diaminobenzol; N-Benzo[1,3]-dioxol-5-ylmethyl-1,4-diamino-benzol; N-{4-[(4-Aminophenylamino)-methyl]-phenyl}-acetamid und N-((4-Methoxyphenyl)-methyl)-1,4-diaminobenzol sowie deren physiologisch verträglichen Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die N-Benzyl-p-phenylendiamin-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy 5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)aminol-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethyl-amino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoe-säure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion in Betracht.

Obwohl die vorteilhaften Eigenschaften der hier beschriebenen Verbindungen der Formel (I) es nahelegen, diese als alleinige Entwicklersubstanz zu verwenden, ist es selbstverständlich auch möglich, diese Verbindungen gemeinsam mit bekannten Entwicklersubstanzen, wie zum Beispiel 1,4-Diaminobenzol, 2,5-Diaminotoluol, 2,5-Diaminophenylethanol, 4-Aminophenol und seinen Derivaten (beispielsweise 4-Amino-3-methylphenol), 4,5-Diamino-1-benzyl-1H-pyrazol, 4,5-Diamino-1-((4'-methylbenzyl)-1H-pyrazol, 4,5 Diamino-1H-pyrazol, 4,5-Diamino-1-(4'-methoxybenzyl)-1 H-pyrazol, 4,5- Diamino-1-(3'-methoxy-benzyl)-1H-pyrazol, 4,5-Diamino-1-(4'-chlor-benzyl)-1H-pyrazol, 4,5-Diamino-1-((4'-methylphenyl)-1H-pyrazol, 4,5-Diamino-1-(4'-methoxy-phenyl)-1H-pyrazol, 4,5- Diamino-1-(3'-methoxy-phenyl)-1H-pyrazol, 4,5-Diamino-1-(4'-chlorphenyl)-1H-pyrazol, 4,5-Diamino-1-(2-hydroxyethyl)-1H-pyrazol, 4,5-Diamino-1-methyl-1 H-pyrazol, 4,5-Diamino-1-ethyl-1H-pyrazol, 4-Amino-1-((4-methoxyphenyl)methyl)-5-(methylamino)-1H-pyrazol, 4-Amino-5-((2-hydroxyethyl)amino)-1-(phenylmethyl)-1H-pyrazol, 4,5-Diamino-1-methyl-3-phenyl-1 H-pyrazol, 4,5-Diamino-1-(2-hydroxyethyl)-3-phenyl-1H-pyrazol, 4,5-Diamino-1,3-dimethyl-1H-pyrazol, 4,5-Diamino-3-methyl-1-phenyl-1H-Pyrazol, 4,5-Diamino-1-(1-isopropyl)-1 H-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicldersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß (beispielsweise in einem Verhältnis (Kuppler : Entwickler) von 1:2 bis 1:0,5) vorhanden sind.
Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxynaphthalin-1-sulfonsäure-Natriumsalz (C.I. 14 805) und Dispersionsfarbstoffe wie beispielsweise 1,4-Diaminoanthrachinon und 1,4,5,8-Tetraamino-antrachinon, enthalten. Die vorgenannten Farbkomponenten können in dem erfindungsgemäßen Färbemittel in einer Menge von etwa 0,1 bis 4 Gewichtsprozent enthalten sein.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem erfindungsgemäßen Färbemittel, falls dieses zur Färbung von Haaren verwendet werden soll, noch weitere für kosmetische Mittel übliche Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.
Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5 Gewichtsprozent

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 50 bis 200 Gramm, dieses Gemisches auf das Haar auf. Das nach dem Vermischen mit dem Oxidationsmittel erhaltene gebrauchsfertige Oxidationshaarfärbemittel hat vorzugsweise einen pH-Wert von 6,5 bis 11,5.
Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12-prozentigen, vorzugsweise 6-prozentigen, wässrigen Lösung, aber auch Luftsauerstoff in Betracht. Wird eine 6-prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an N-Benzyl-p-phenylendiamin-Derivaten der Formel (I) als Entwicklersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, daß dieses Mittel insbesondere auch eine Anfärbung von ergrauten, chemisch nicht vorgeschädigten Haaren problemlos und mit guter Deckkraft ermöglicht.

Die Herstellung der erfindungsgemäßen N-Benzyl-p-phenylendiamin - Derivate der Formel (I) kann unter Verwendung von bekannten Syntheseverfahren, beispielsweise dem in den Ausführungsbeispielen beschriebenen Verfahren, erfolgen.

Die N-Benzyl-p-phenylendiamin-Derivate der Formel (I) sind gut in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der hier beschriebenen Oxidationsfärbemitteln, auf.

Ein weiterer Gegenstand der vorliegenden Erfindung sind neue N-Benzyl-p-phenylendiamin-Derivate der allgemeinen Formel (I), wobei (i) **R4** nicht gleich einer Methylgruppe, einer Hydroxygruppe, einer Dimethylaminogruppe, einem Bromatom oder einem Chloratom ist, wenn gilt **R1=R2=R3=R5=** Wasserstoff, und (ii) mindestens einer der verbleibenden Reste **R1** bis **R5** von Wasserstoff verschieden ist, wenn **R3** und **R4** bzw. **R4** und **R5** eine Methoxygruppe darstellen, und (iii) **R4** nicht gleich einer Hydroxygruppe ist, wenn **R1** und **R2** ein Wasserstoffatom darstellen und **R3** und **R5** eine t-Butylgruppe bedeuten; oder deren physiologisch verträgliche, wasserlösliche Salze.

Die nachfolgenden Beispiele sollen den Gegenstand der Erfindung näher erläutern, ohne ihn darauf zu beschränken.

### Beispiele

### Beispiele 1: Synthese von N-Benzyl-1,4-diamino-benzolen

0,031 g (0,15 mmol) N-(4-Aminophenyl)-carbaminsäure-tert-butylester und 0,10 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50 °C erwärmt.
Der Niederschlag wird abfiltriert, zweimal mit 1 ml Ethanol gewaschen und sodann getrocknet.
**a.** N-((4-(2-Hydroxyethoxy)-phenyl)methyl)-1,4-diaminobenzol Hydrochlorid
   Verwendeter Aldehyd: 4-(2-Hydroxyethoxy)-benzaldehyd
   Ausbeute: 0,025 g (75% der Theorie)
   Massenspektrum: MH+ 259(100)
**b.** N-{4-[(4-Amino-phenylamino)-methyl]-phenyl}-acetamid Hydrochlorid
   Verwendeter Aldehyd: 4-Acetamino-benzaldehyd
   Ausbeute: 0,025 g (76% der Theorie)
   Massenspektrum: MH+ 256(100)
**c.** 4-[(4-Amino-phenylamino)-methyl]-2,6-dimethyl-phenol Hydrochlorid
   Verwendeter Aldehyd: 2,6-Dimethyl-4-hydroxy-benzaldehyd
   Ausbeute: 0,025 g (79% der Theorie)
   Massenspektrum: MH+ 243(100)
**d.** N-Benzo[1,3]dioxol-5-ylmethyl-1,4-diamino-benzol Hydrochlorid
   Verwendeter Aldehyd: 3,4-Methylendioxy-benzaldehyd
   Ausbeute: 0,025 g (79% der Theorie)
   Massenspektrum: MH+ 316(100)
**e.** N-((4-Hydroxyphenyl)-methyl)-1,4-diaminobenzol
   Verwendeter Aldehyd: 4-Hydroxy-benzaldehyd
   Ausbeute: 0.025 g (100% der Theorie)
   Massspketren: MH+ 215(100)
**f.** N-((4-Methoxyphenyl)methyl)-1,4-diaminobenzol Hydrochlorid
   Verwendeter Aldehyd: 4-Methoxy-benzaldehyd
   Ausbeute: 0,025 g (83% der Theorie)
   Massenspektrum: MH⁺ 229(100)
**g.** N-(4-Pyrrolidin-1-yl-benzyl)-1,4-diamino-benzol (nicht erfindungsgemäss)
   Verwendeter Aldehyd: 4-Pyrrolidino-benzaldehyd
   Ausbeute: 10 g (30% der Theorie)
**h.** 2-[{4-[(4-Amino-phenylamino)-methyl]-phenyl}-(2-hydroxyethyl)amino]-ethanol Hydrochlorid
   Verwendeter Aldehyd: 4-(Bis(2-Hydroxyethyl)amino)-benzaldehyd
   Ausbeute: 0,025 g (60% der Theorie)
**i.** N-(4-Nitro-benzyl)-1,4-diamino-benzol Hydrochlorid (nicht erfindungsgemäss)
   Verwendeter Aldehyd: 4-Nitro-benzaldehyd
   Ausbeute: 0,025 g (79% der Theorie)
   Massenspektrum: MH+ 244(20)
**j.** N-(4-Dimethylamino-benzyl)-1,4-diamino-benzol
   Verwendeter Aldehyd: 4-Dimethylamino-benzaldehyd
   Ausbeute: 0,025 g (100% der Theorie)
   Massenspektrum: MH+ 242(25)
**k.** N-(2,4-Dinitro-benzyl)-1,4-diamino-benzol Hydrochlorid (nicht erfindungsgemäss)
   Verwendeter Aldehyd: 2,4-Dinitro-benzaldehyd
   Ausbeute: 0,025 g (69% der Theorie)
   Massenspektrum: MH+ 289(70)

### Beispiel 2: Synthese von N¹-Benzyl-1,4-diamino-2-methyl-benzolen und N⁴-Benzyl-1,4-diamino-2-methyl-benzolen

0,033 g (0,15 mmol) eine Mischung aus N-(4-Amino-2-methyl-phenyl)-carbaminsäure-tert-butylester und von N-(4-Amino-3-methyl-phenyl)-carbaminsäure-tert-butylester und 0,1 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50 °C erwärmt. Der Niederschlag wird abfiltriert, zweimal mit 1 ml Ethanol gewaschen und sodann getrocknet.
**a.** N¹-(4-Methoxy-benzyl)-2-methyl-1,4-diamino-benzol und
   N¹-(4-Methoxy-benzyl)-3-methyl-1,4-diamino-benzol Hydrochlorid
   Verwendete Aldehydderivat: 4-Methoxy-benzaldehyd
   Ausbeute: 0,025 g (39% der Theorie)
   Massenspektrum: MH+ 243(100)
**b.** 2-Methyl-N¹-(4-nitro-benzyl)-1,4-diamino-benzol Hydrochlorid und
   3-Methyl-N¹-(4-nitro-benzyl)-1,4-diamino-benzol Hydrochlorid
   (nicht enfindungsgemäss)
   Verwendeter Aldehyd: 4-Nitro-benzaldehyd
   Ausbeute: 0,025 g (37% der Theorie)
   Massenspektrum: MH+ 258(100)
**c.** N-{4-[(4-Amino-2-methyl-phenylamino)-methyl]-phenyl}-acetamid
   Hydrochlorid und N-{4-[(4-Amino-3-methyl-phenylamino)-methyl]-phenyl}-acetamid Hydrochlorid
   Verwendeter Aldehyd: 4-Acetamido-benzaldehyd
   Ausbeute: 0,025 g (36% der Theorie)
   Massenspektrum: MH+ 270(100)
**d.** 4-[(4-Amino-2-methyl-phenylamino)-methyl]-phenol Hydrochlorid und
   4-[(4-Amino-3-methyl-phenylamino)-methyl]-phenol Hydrochlorid
   Verwendeter Aldehyd: 4-Hydroxy-benzaldehyd
   Ausbeute: 0,025 g (41% der Theorie)
   Massenspektrum: MH+ 229(100)
**e.** N¹-(4-Dimethylamino-benzyl)-2-methyl-1,4-diamino-benzol und
   N¹-(4-Dimethylamino-benzyl)-3-methyl-1,4-diamino-benzol
   Verwendeter Aldehyd: 4-Dimethylamino-benzaldehyd
   Ausbeute: 0,025 g (48% der Theorie)
   Massenspektrum: MH- 254(100)

### Beispiele 3: Synthese von N¹-Benzyl-1,4-diamino-2-(2-hydroxyethyl)-benzolen und N⁴-Benzyl-1,4-diamino-2-(2-hydroxyethyl)-benzolen

0,038 g (0,15 mmol) eine Mischung von N-(4-Amino-2-(2-hydroxyethyl)-phenyl)-carbaminsäure-tert-butylester und von N-(4-Amino-3-(2-hydroxyethyl)-phenyl)-carbaminsäure-tert-butylester und 0,1 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9 molaren ethanolische Salzsäurelösung auf 50 °C erwärmt.
Der Niederschlag wird abfiltriert, zweimal mit 1 ml Ethanol gewaschen und sodann getrocknet.
**a.** 2-[5-Amino-2-(4-nitro-benzylamino)-phenyl]-ethanol Hydrochlorid und 2-[6-Amino-3-(4-nitro-benzylamino)-phenyl]-ethanol Hydrochlorid (nicht enfindungsgemäss)
   Verwendeter Aldehyd: 4-Nitro-benzaldehyd
   Ausbeute: 0,025 g (34% der Theorie)
   Massenspektrum: MH+ 288(100)
**b.** 2-[5-Amino-2-(4-methoxy-benzylamino)-phenyl]-ethanol Hydrochlorid und 2-[6-Amino-3-(4-methoxy-benzylamino)-phenyl]-ethanol Hydrochlorid
   Verwendeter Aldehyd: 4-Methoxy-benzaldehyd
   Ausbeute: 0,025 g (35% der Theorie)
   Massenspektrum: MH+ 273(100)
c. 2-[(4-{[4-Amino-2-(2-hydroxyethyl)-phenylamino]-methyl}-phenyl)-(2-hydroxyethyl)-amino]-ethanol und 2-[(4-{[4-Amino-3-(2-hydroxyethyl)-phenylamino]-methyl}-phenyl)-(2-hydroxyethyl)-amino]-ethanol
   Verwendeter Aldehyd: 4-Bis(2-hydroxyethyl)amino-benzaldehyd
   Ausbeute: 15 g (25% der Theorie)
**d.** 2-[5-Amino-2-(4-dimethylamino-benzylamino)-phenyl]-ethanol und 2-[6-Amino-3-(4-dimethylamino-benzylamino)-phenyl]-ethanol
   Verwendeter Aldehyd: 4-Dimethylamino-benzaldehyd
   Ausbeute: 0,025 g (42% der Theorie)

### Beispiele 4 bis 23: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8-prozentige wässrige Lösung) |
| 1,0 g | Ammoniak (22-prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6-prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

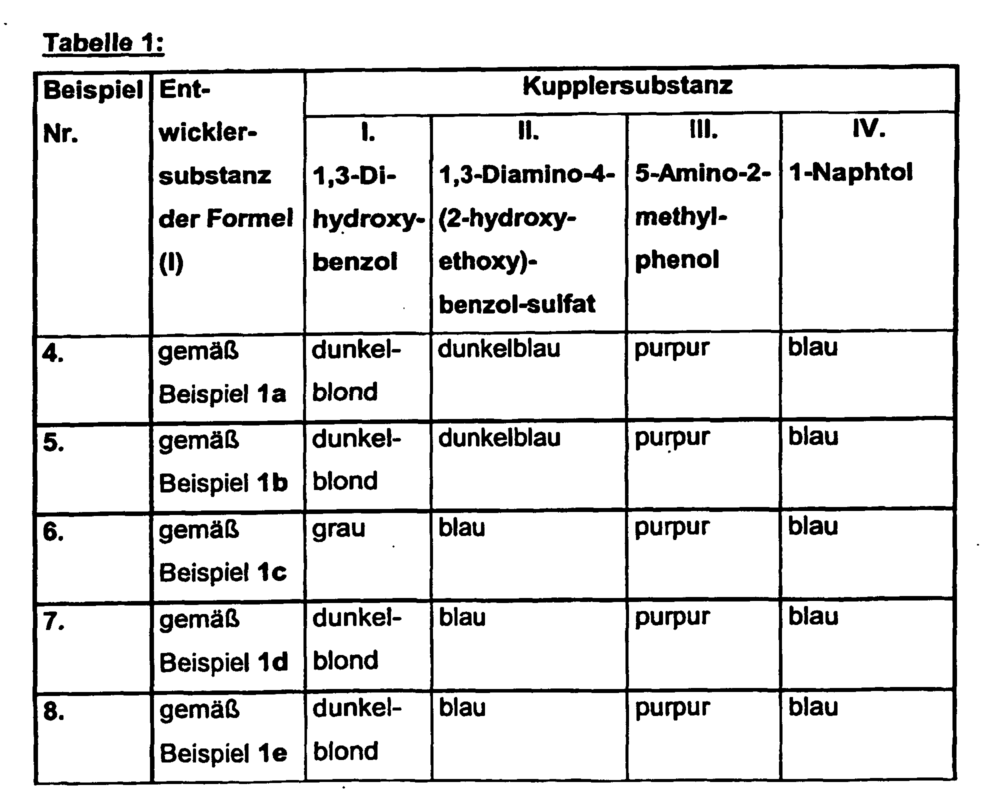

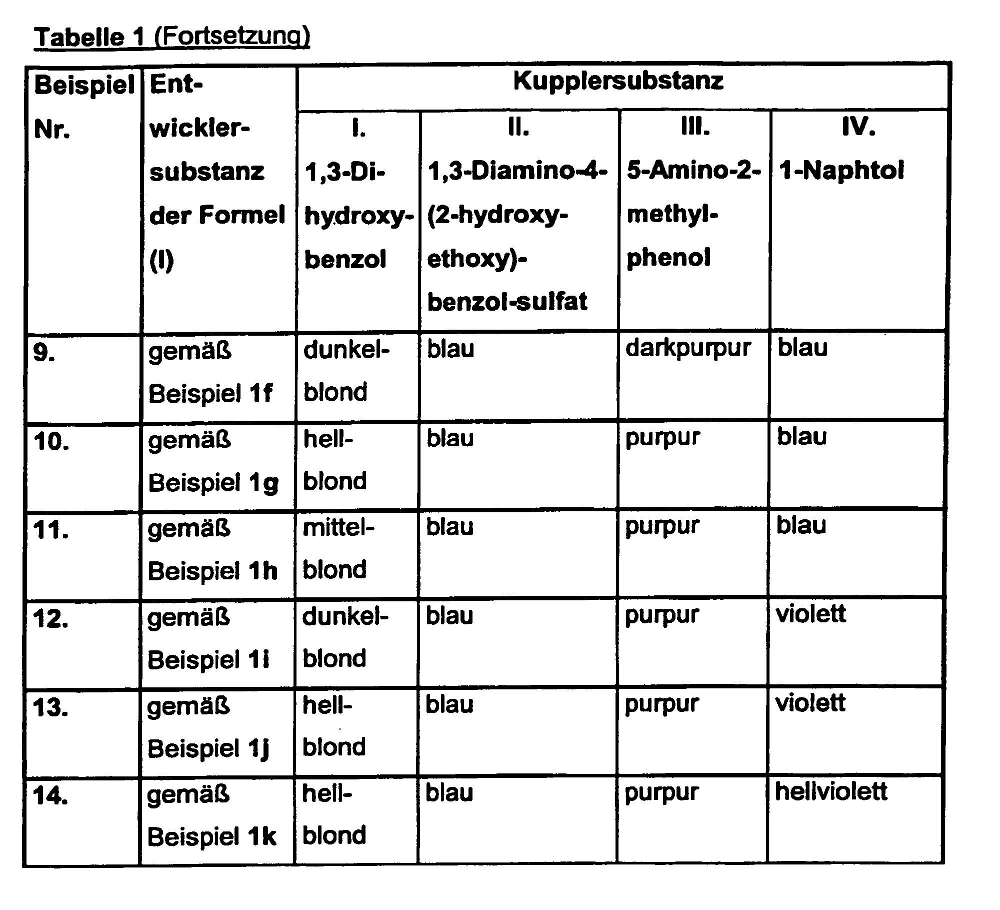

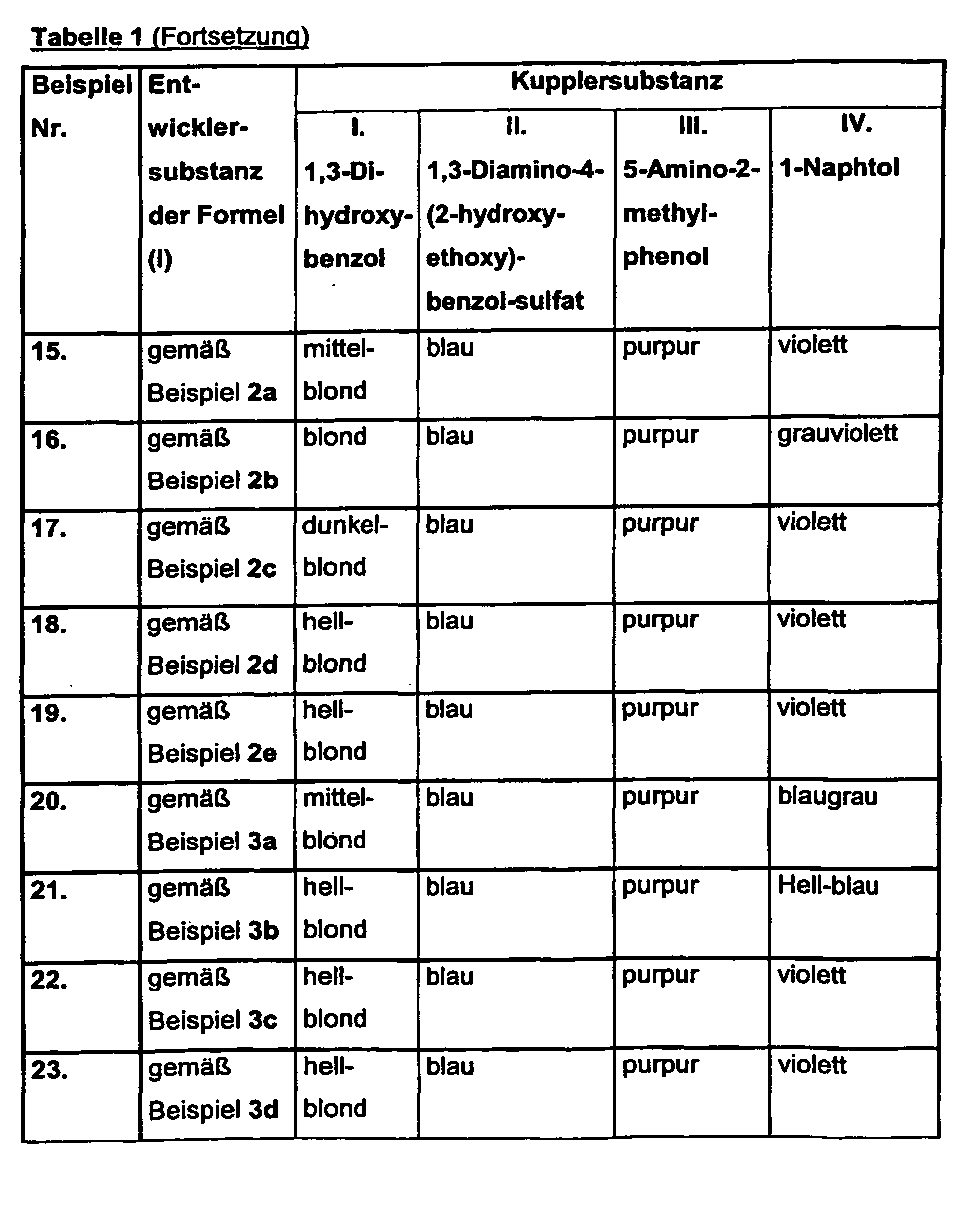

### Beispiele 24 bis 73: Haarfärbemittel

Es werden Haarfärbelösungen der folgenden Zusammensetzung hergestellt:

| | |
|---|---|
| X g | N-(Benzyl)-1,4-diamino-benzol (Entwicklersubstanz **E3** bis **E7** der Formel (I) gemäß Tabelle 2) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,0 g | Kaliumoleat (8-prozentige wässrige Lösung) |
| 10,0 g | Ammoniak (22-prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

### Beispiele 74 bis 103: Haarfärbemittel

Es werden cremeförmige Farbträgermassen der folgenden Zusammensetzung hergestellt:

| | |
|---|---|
| X g | N-(Benzyl)-1,4-Diamino-benzol (Entwicklersubstanz E3 bis **E7** der Formel (I) gemäß Tabelle 2) |
| U g | Entwicklersubstanz **E8** bis **E15** gemäß Tabelle 2 |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28-prozentige wässrige Lösung |
| 3,0 g | Ammoniak, 22-prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6-prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

**Tabelle 2:**

| **Entwicklersubstanzen** | |
|---|---|
| **E3** | N-((4-(2-Hydroxyethoxy)-phenyl)methyl)-1,4-diaminobenzol Hydrochlorid |
| **E4** | N-((4-Methoxyphenyl)methyl)-1,4-diaminobenzol Hydrochlorid |
| **E5** | N-{4-[(4-Amino-phenylamino)-methyl]-phenyl}-acetamid Hydrochlorid |
| **E6** | N-((4-Hydroxyphenyl)-methyl)-1,4-diaminobenzol |
| **E7** | N-Benzo[1,3]dioxol-5-ylmethyl-1,4-diamino-benzol Hydrochlorid |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzolhydrochlorid |
| **K35** | 3,4-Methylendioxy-phenol |
| **K36** | 2-Amino-5-methyl-phenol |

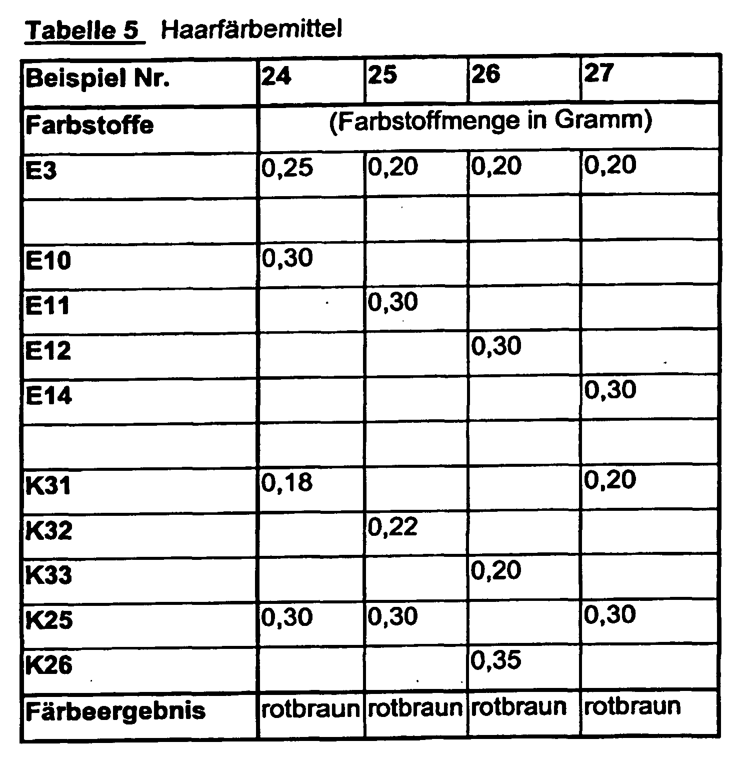

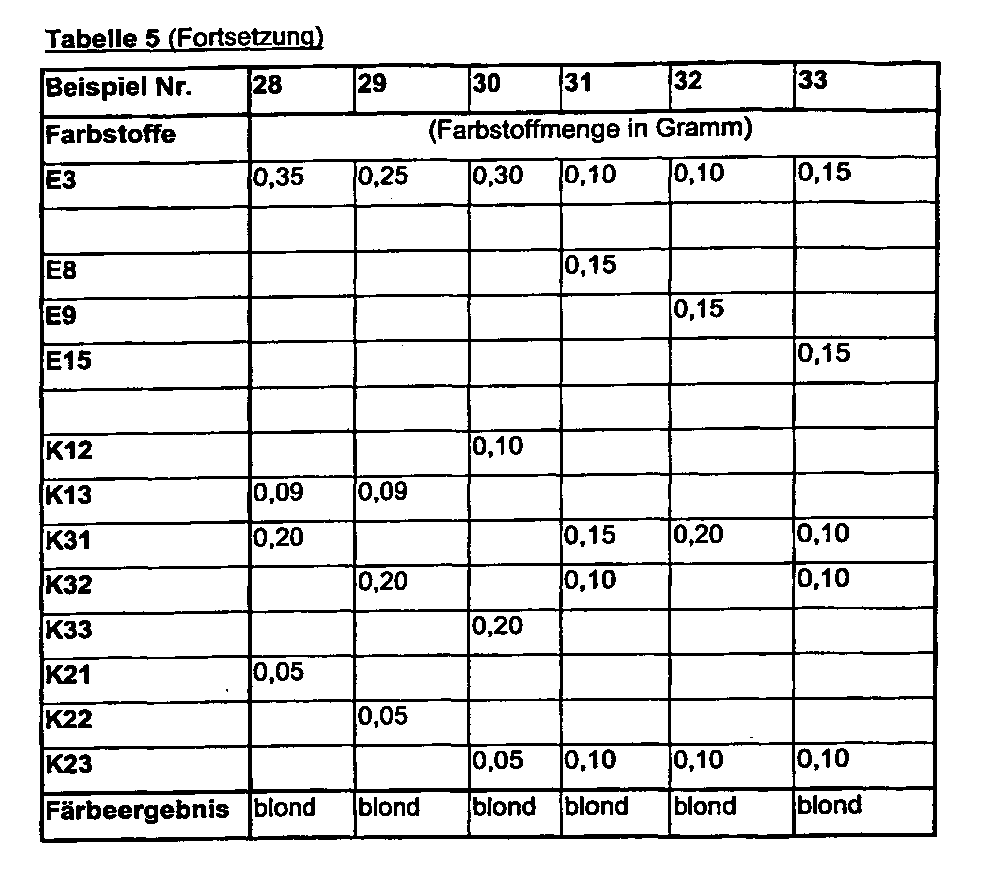

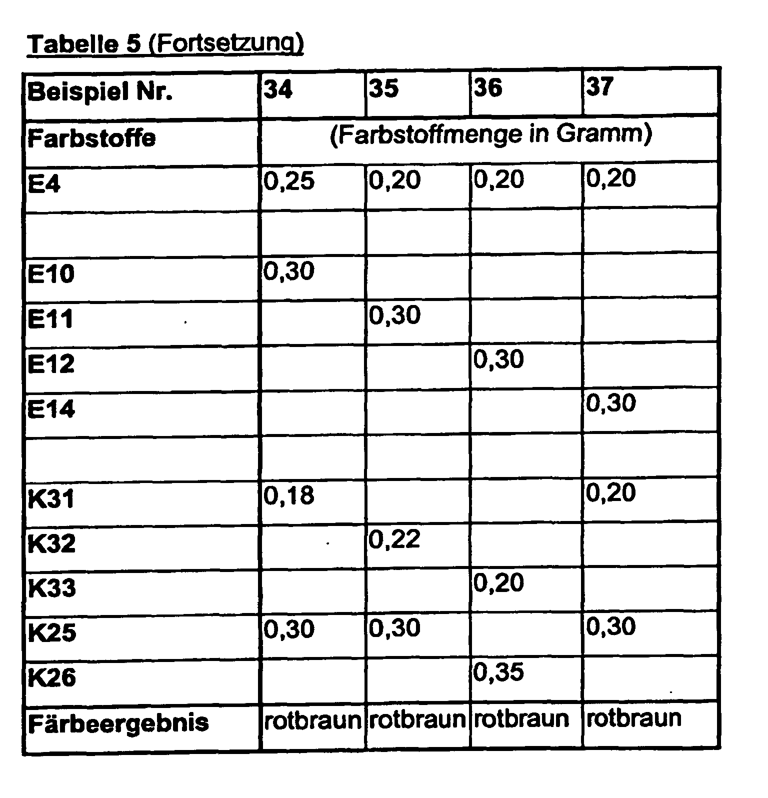

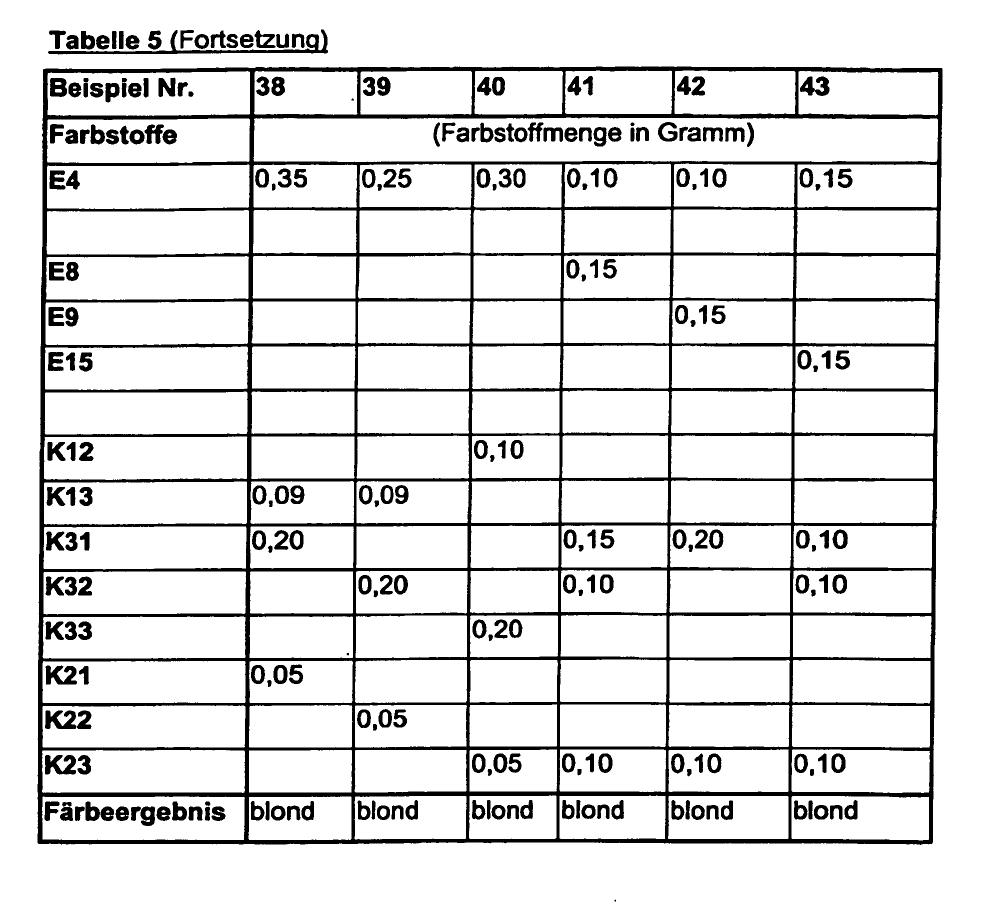

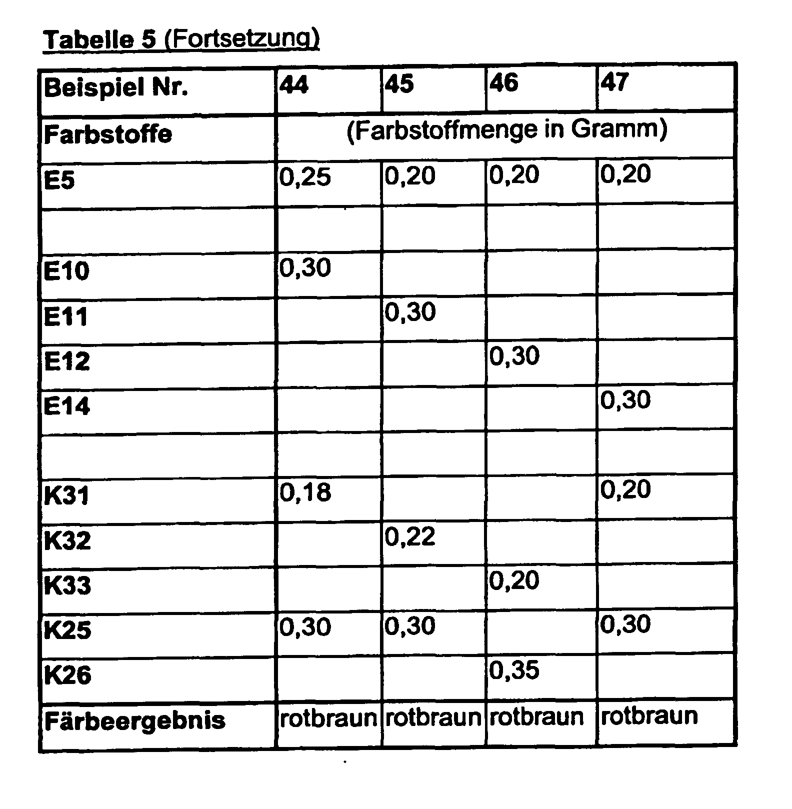

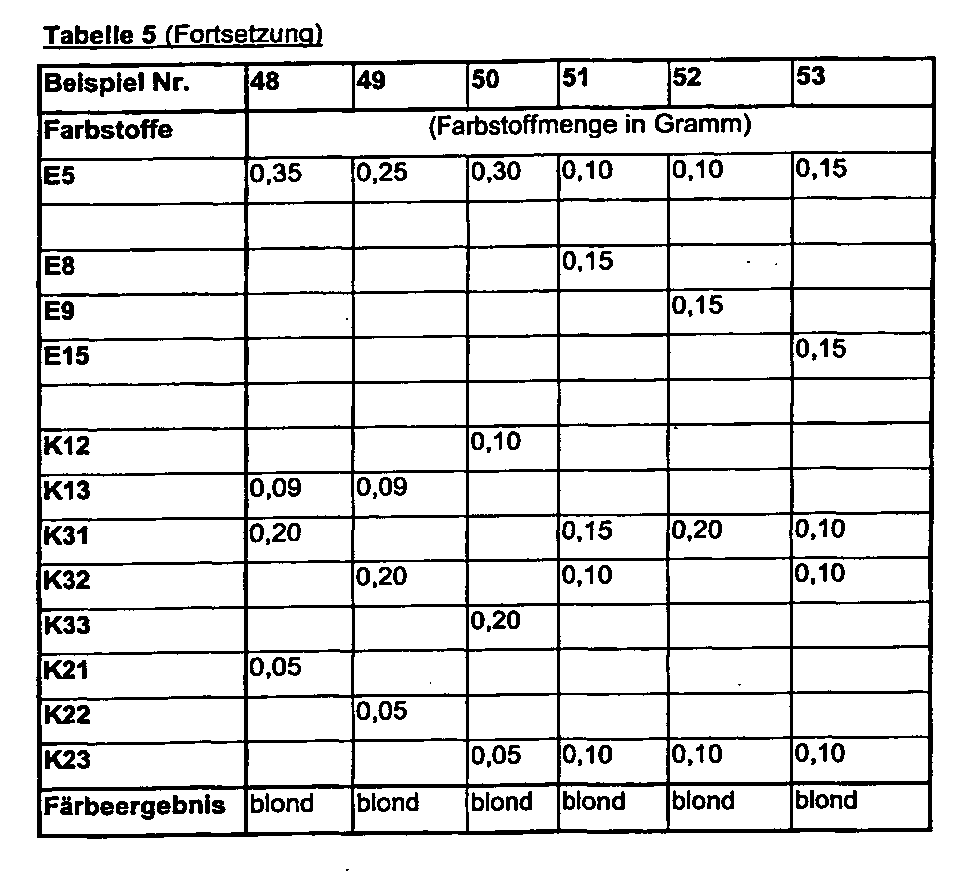

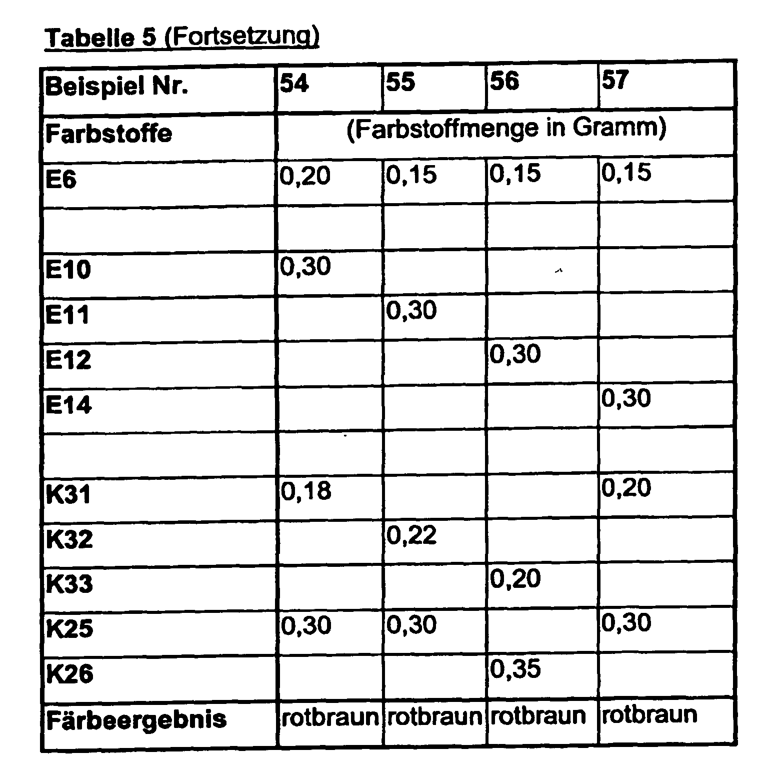

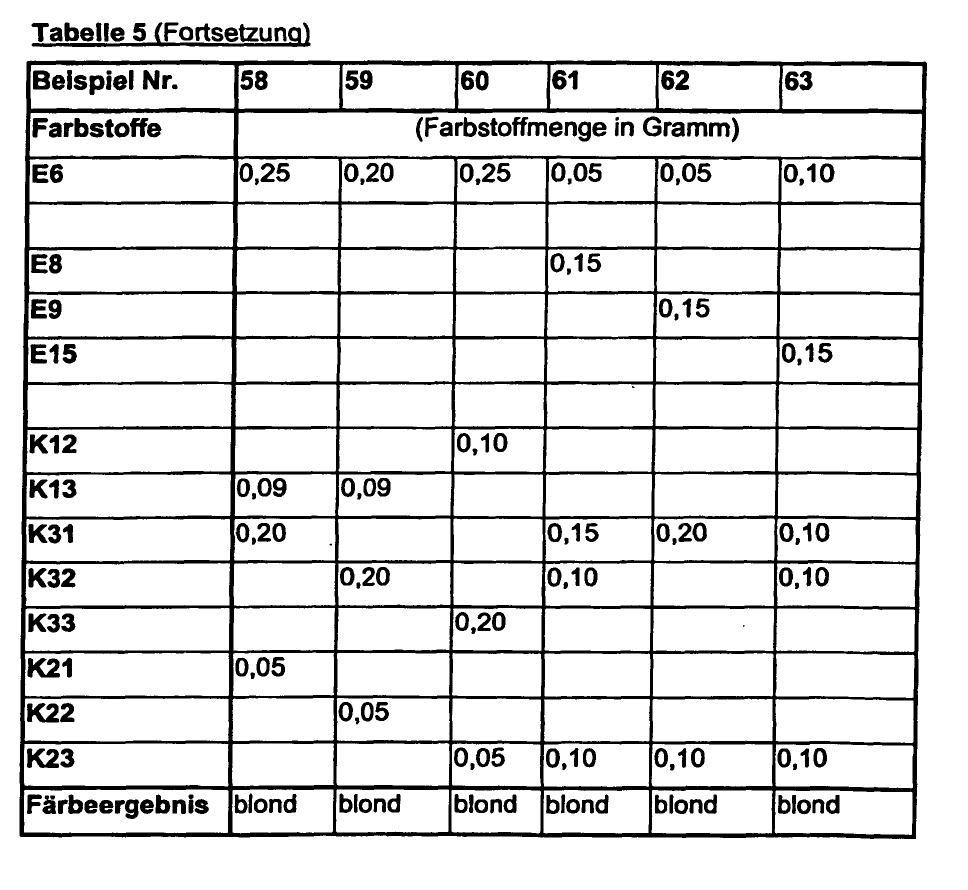

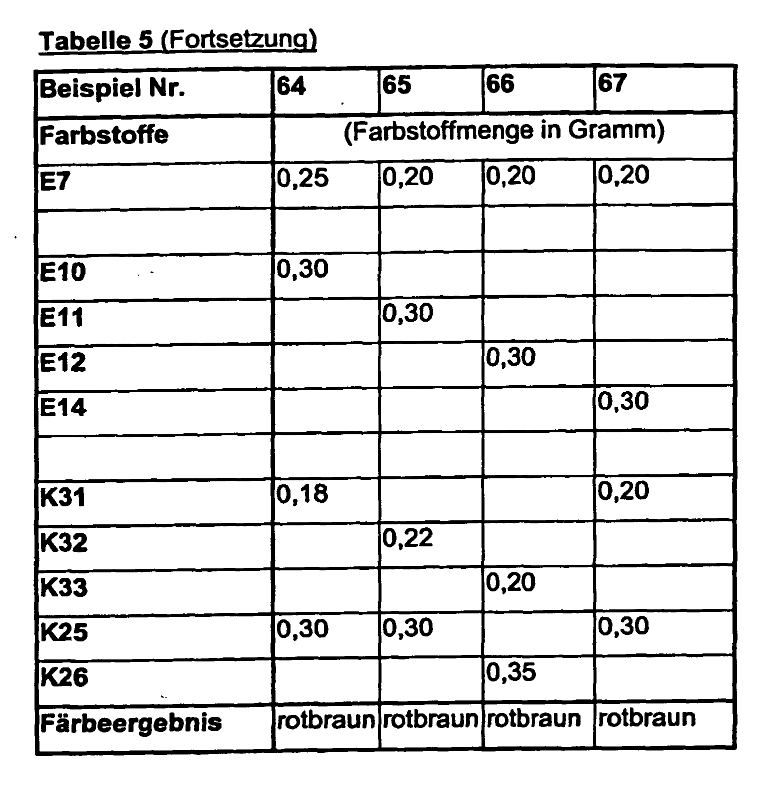

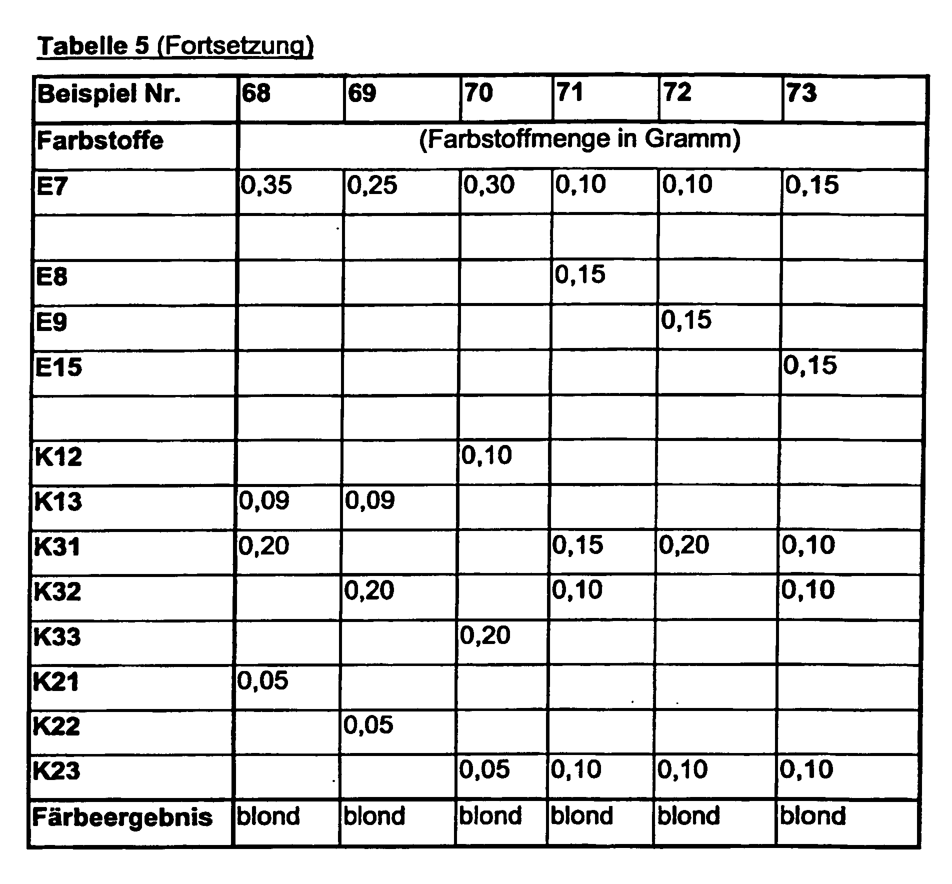

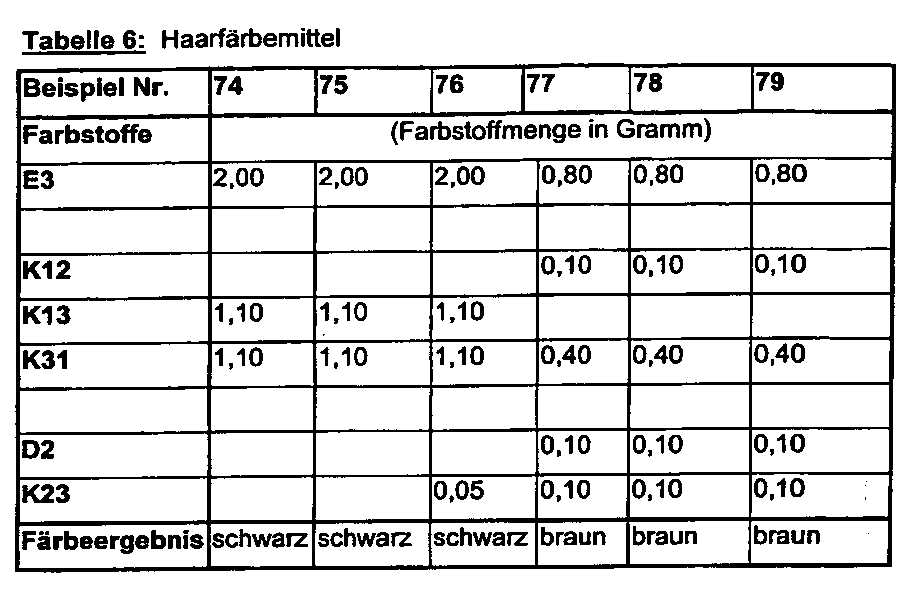

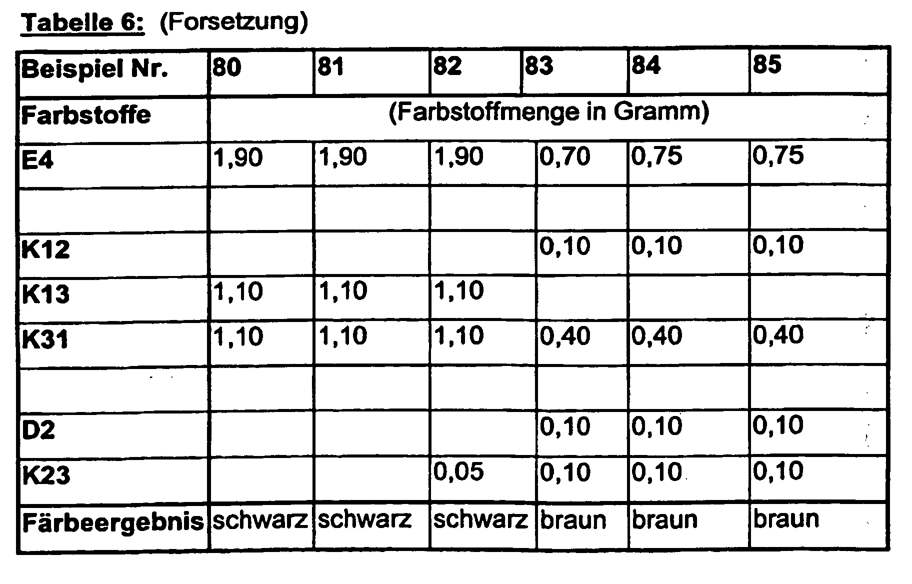

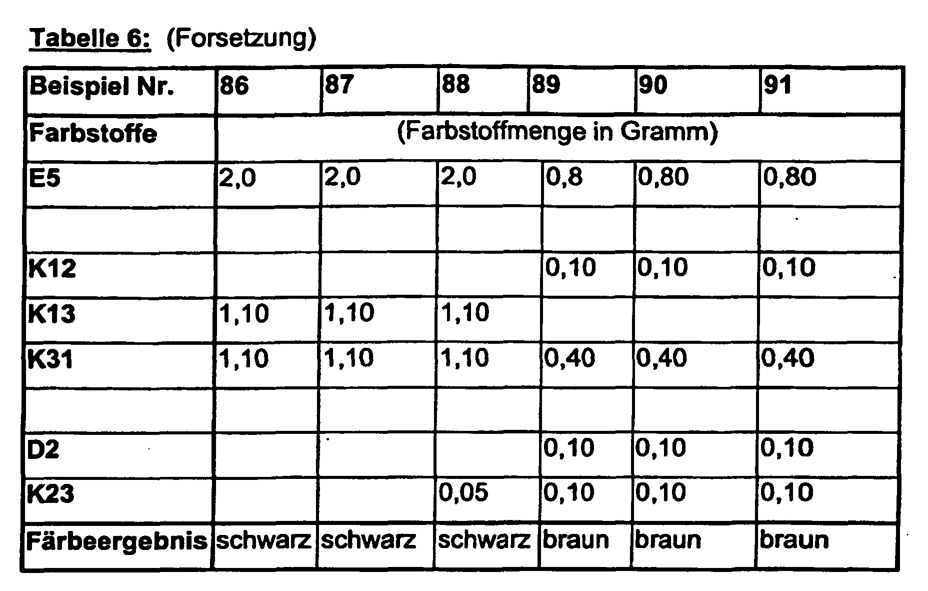

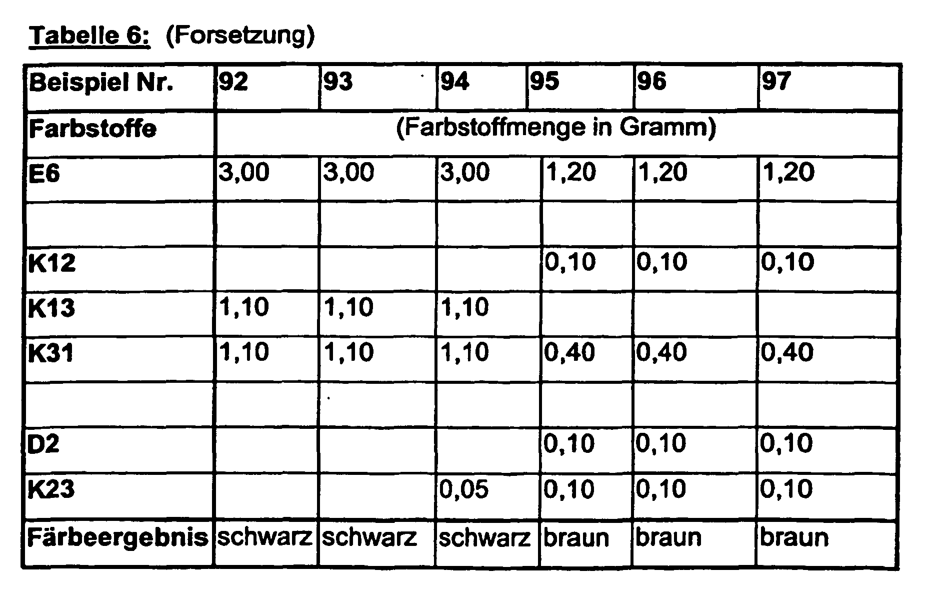

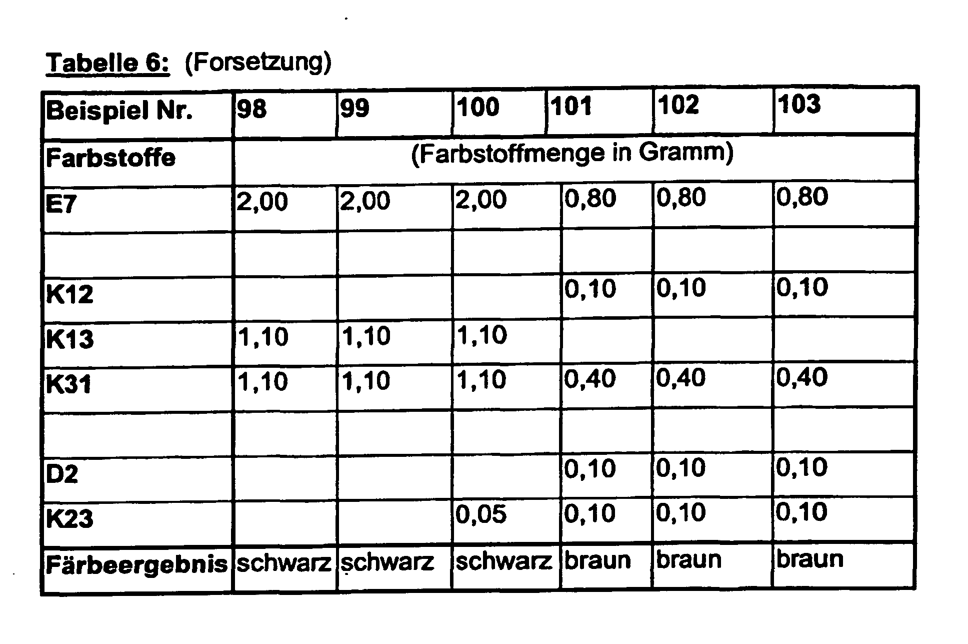

Alle in der vorliegenden Anmeldung enthaltenen Prozentangaben stellen soweit nicht anders angegeben Gewichtsprozente dar.

## Patentansprüche

1. N-Benzyl-p-phenytendiamin-Derivate der allgemeinen Formel (I) oder deren physiologisch verträgliche, wasserlösliche Salze, 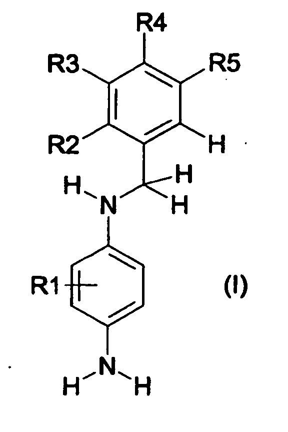 worin
**R1** gleich Wasserstoff, einer (C₁-C₄)-Alkylgruppe oder einer Hydroxy-(C₁-C₄)-alkylgruppe ist ;
R2 gleich Wasserstoff, einem Halogenatom (F, Cl, Br, J), einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy-(C₁-C₄)-alkyl)-aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder eine Morpholinogruppe ist;
R3 Wasserstoff, ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
R4 ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt, oder R3 und R4 gemeinsam eine -O-CH2-O-Brücke bilden;
R5 gleich Wasserstoff, einer Hydroxygruppe oder einer (C₁-C₆)-Alkylgruppe ist; unter der Bedingung, dass
(i) R4 nicht gleich einer Methylgruppe, einer Hydroxygruppe, einer Dimethylaminogruppe, einem Bromatom oder einem Chloratom ist, wenn gilt **R1=R2=R3=R5=** Wasserstoff, und (ii) mindestens einer der verbleibenden Reste **R1** bis **R5** von Wasserstoff verschieden ist, wenn **R3** und **R4** bzw. **R4** und **R5** eine Methoxygruppe darstellen, und (iii) **R4** nicht gleich einer Hydroxygruppe ist, wenn **R1** und **R2** ein Wasserstoffatom darstellen und **R3** und **R5** eine t-Butylgruppe bedeuten.

2. Mittel zur oxidativen Färbung von Keratinfasern auf der Basis einer Entwicklersubstanz-Kupplersubstanz-Kombination, **dadurch gekennzeichnet, dass** es als Entwicklersubstanz mindestens ein N-Benzyl-p-phenylendiamin-Derivat der allgemeinen Formel (I) oder dessen physiologisch verträgliche, wasserlösliche Salze enthält, 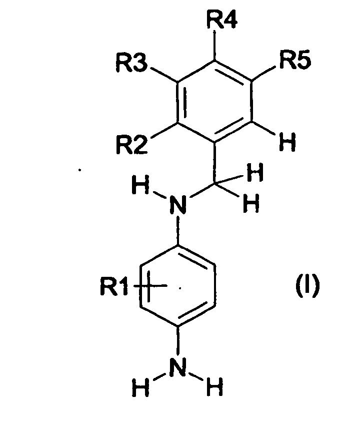 worin
**R1** gleich Wasserstoff, einer (C₁-C₄)-Alkylgruppe oder einer Hydroxy-(C₁-C₄)-alkylgruppe ist ;
R2 gleich Wasserstoff, einem Halogenatom (F, Cl, Br, J), einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy-(C₁-C₄)-alkyl)-aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder eine Morpholinogruppe ist;
R3 Wasserstoff, ein Halogenatom, eine Hydroxygruppe, eine (C₁-C₄)-Alkoxygruppe, eine Hydroxy-(C₁-C₄)-alkoxy-gruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
**R4** ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine (C₁-C₆)-Alkylaminogruppe, eine Di-(C₁-C₆)-alkylaminogruppe, eine Di-(hydroxy-(C₁-C₄)-alkyl)-aminogruppe, eine Hydroxy-(C₁-C₄)-alkylaminogruppe, eine Trifluormethangruppe, eine Acetamido-gruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt; oder **R3** und **R4** gemeinsam eine -O-CH2-O-Brücke bilden;
**R5** gleich Wasserstoff, einer Hydroxygruppe oder einer (C₁-C₆)-Alkylgruppe ist.

3. Mittel nach Anspruch 2, **dadurch gekennzeichnet, dass** die Verbindung der Formel (I) ausgewählt ist aus der Gruppe bestehend aus N-((4-Hydroxyphenyl)-methyl)-1,4-diamino-benzol; N-((4-Hydroxy-3,5-dimethyl-phenyl)methyl)-1,4-diaminobenzol; N-((4-(2-Hydroxyethoxy)-phenyl)methyl)-1,4-diaminobenzol; N-Benzo[1,3]-dioxol-5-ylmethyl-1,4-diamino-benzol; N-{4-[(4-Aminophenylamino)-methyl]-phenyl}-acetamid und N-((4-Methoxyphenyl)-methyl)-1,4-diaminobenzol sowie den physiologisch verträglichen Salzen dieser Verbindungen.

4. Mittel nach Anspruch 2 oder 3, **dadurch gekennzeichnet, dass** das N-Benzyl-p-phenylendiamin-Derivat der Formel (I) in einer Menge von 0,005 bis 20 Gewichtsprozent enthalten ist.

5. Mittel nach einem der Ansprüche 2 bis 4, **dadurch gekennzeichnet, dass** es einen pH-Wert von 6,5 bis 11,5 aufweist.

6. Mittel nach einem der Ansprüche 2 bis 5, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylaminophenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)-amino]-1,3-benzodioxol, 6-Brom-1-hydroxy-3,4-methylendioxy-benzol, 3,4-Diamino-benzoe-säure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion.

7. Mittel nach einem der Ansprüche 2 bis 6, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

8. Mittel nach einem der Ansprüche 2 bis 7, **dadurch gekennzeichnet, dass** es zusätzlich mindestens einen direktziehenden Farbstoff enthält.

9. Mittel nach einem der Ansprüche 2 bis 8, **dadurch gekennzeichnet, dass** es ein Haarfärbemittel ist.

## Claims

1. N-Benzyl-p-phenylenediamine derivatives of the general formula (I) or physiologically compatible, water-soluble salts thereof, 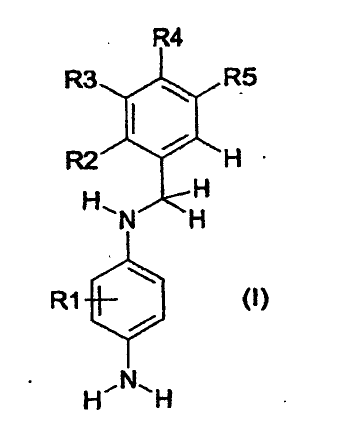 in which
**R1** is hydrogen, a (C₁-C₄)-alkyl group or a hydroxy-(C₁-C₄)-alkyl group;
**R2** is hydrogen, a halogen atom (F, Cl, Br, I), a cyano group, a (C₁-C₄)-alkoxy group, a hydroxy-(C₁-C₄)-alkoxy group, a (C₁-C₆)-alkyl group, a (C₁-C₄)-alkyl thioether group, a mercapto group, a nitro group, a (C₁-C₄)-alkylamino group, a di(C₁-C₄)-alkylamino group, a di (hydroxy (C₁-C₄)-alkyl) amino group, a (hydroxy(C₁-C₄)-alkyl)amino group, a trifluoromethane group, a -C(O)CH₃ group, a -C(O)CF₃ group, a -Si(CH₃)₃ group, a hydroxy(C₁-C₄)-alkyl group, a dihydroxy(C₃-C₄)-alkyl group or a morpholino group;
**R3** is hydrogen, a halogen atom, a hydroxy group, a (C₁-C₄)-alkoxy group, a hydroxy(C₁-C₄)-alkoxy group, a (C₁-C₆)-alkyl group, a (C₁-C₄)-alkyl thioether group, a mercapto group, a (C₁-C₆)-alkylamino group, a di(C₁-C₆)-alkylamino group, a di(hydroxy(C₁-C₄)-alkyl)amino group, a hydroxy(C₁-C₄)-alkylamino group, a trifluoromethane group, an acetamido group, a -C(O)CH₃ group, a -C(O)CF₃ group, a -Si(CH₃)₃ group, a hydroxy(C₁-C₄)-alkyl group or a dihydroxy(C₃-C₄)-alkyl group;
**R4** is a halogen atom, a hydroxy group, a (C₁-C₄)-alkoxy group, a hydroxy(C₁-C₄)-alkoxy group, a (C₁-C₆)-alkyl group, a (C₁-C₄)-alkyl thioether group, a mercapto group, a (C₁-C₆)-alkylamino group, a di (C₁-C₆)-alkylamino group, a di(hydroxy(C₁-C₄)-alkyl)amino group, a hydroxy(C₁-C₄)alkylamino group, a trifluoromethane group, an acetamido group, a -C(O)CH₃ group, a -C(O)CF₃ group, a -Si(CH₃)₃ group, a hydroxy(C₁-C₄)-alkyl group or a dihydroxy(C₃-C₄)-alkyl group, or **R3** and **R4** together form a -O-CH2-O bridge;
**R5** is hydrogen, a hydroxy group or a (C₁-C₆)-alkyl group; under the condition that
(i) **R4** is not a methyl group, a hydroxy group, a dimethylamino group, a bromine atom or a chlorine atom when **R1 = R2 = R3 = R5 =** hydrogen, and (ii) at least one of the remaining radicals **R1** to **R5** is different from hydrogen when **R3** and **R4** or **R4** and **R5** are a methoxy group, and (iii) **R4** is not a hydroxy group if **R1** and **R2** are a hydrogen atom and **R3** and **R5** are a t-butyl group.

2. Agent for the oxidative colouring of keratin fibres based on a developer substance-coupler substance combination, **characterized in that** it comprises, as developer substance, at least one N-benzyl-p-phenylenediamine derivative of the general formula (I) or physiologically compatible, water-soluble salts thereof, 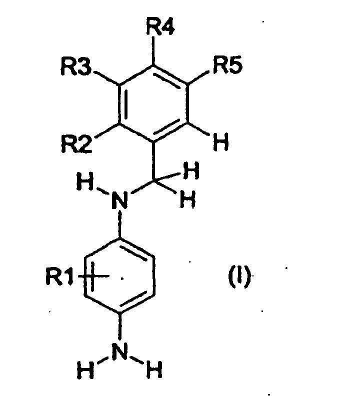 in which
**R1** is hydrogen, a (C₁-C₄)-alkyl group or a hydroxy-(C₁-C₄)-alkyl group;
**R2** is hydrogen, a halogen atom (F, Cl, Br, I), a cyano group, a (C₁-C₄)-alkoxy group, a hydroxy-(C₁-C₄)-alkoxy group, a (C₁-C₆)-alkyl group, a (C₁-C₄)-alkyl thioether group, a mercapto group, a nitro group, a (C₁-C₄)-alkylamino group, a di(C₁-C₄)-alkylamino group, a di (hydroxy(C₁-C₄)-alkyl)amino group, a (hydroxy(C₁-C₄)-alkyl)amino group, a trifluoromethane group, a -C(O)CH₃ group, a -C(O)CF₃ group, a -Si(CH₃)₃ group, a hydroxy(C₁-C₄)-alkyl group, a dihydroxy(C₃-C₄)-alkyl group or a morpholino group;
**R3** is hydrogen, a halogen atom, a hydroxy group, a (C₁-C₄)-alkoxy group, a hydroxy(C₁-C₄)-alkoxy group, a (C₁-C₆)-alkyl group, a (C₁-C₄)-alkyl thioether group, a mercapto group, a (C₁-C₆)-alkylamino group, a di(C₁-C₆)-alkylamino group, a di (hydroxy(C₁-C₄)-alkyl)amino group, a hydroxy(C₁-C₄)-alkylamino group, a trifluoromethane group, an acetamido group, a -C(O)CH₃ group, a -C(O)CF₃ group, a -Si(CH₃)₃ group, a hydroxy(C₁-C₄)-alkyl group or a dihydroxy(C₃-C₄)-alkyl group;
**R4** is a halogen atom, a hydroxy group, a (C₁-C₄)-alkoxy group, a hydroxy(C₁-C₄)-alkoxy group, a (C₁-C₆)-alkyl group, a (C₁-C₄)-alkyl thioether group, a mercapto group, a (C₁-C₆)-alkylamino group, a di (C₁-C₆)-alkylamino group, a di(hydroxy(C₁-C₄)-alkyl)amino group, a hydroxy(C₁-C₄)alkylamino group, a trifluoromethane group, an acetamido group, a -C(O)CH₃ group, a -C(O)CF₃ group, a -Si(CH₃)₃ group, a hydroxy(C₁-C₄)-alkyl group or a dihydroxy(C₃-C₄)-alkyl group; or **R3** and **R4** together form a -O-CH2-O bridge;
**R5** is hydrogen, a hydroxy group or a (C₁-C₆)-alkyl group.

3. Agent according to Claim 2, **characterized in that** the compound of the formula (I) is chosen from the group consisting of N-((4-hydroxyphenyl)methyl)-1,4-diaminobenzene; N-((4-hydroxy-3,5-dimethylphenyl)methyl)-1,4-diaminobenzene; N-((4-(2-hydroxyethoxy)phenyl)methyl)-1,4-diaminobenzene; N-benzo[1,3]dioxol-5-ylmethyl-1,4-diaminobenzene; N-{4-[(4-aminophenylamino)methyl]phenyl}acetamide and N-((4-methoxyphenyl)methyl)-1,4-diaminobenzene and the physiologically compatible salts of these compounds.

4. Agent according to Claim 2 or 3, **characterized in that** the N-benzyl-p-phenylenediamine derivative of the formula (I) is present in an amount of from 0.005 to 20% by weight.

5. Agent according to one of Claims 2 to 4,
**characterized in that** it has a pH of from 6.5 to 11.5.

6. Agent according to one of Claims 2 to 5, **characterized in that** the coupler substance is chosen from the group consisting of 2,6-diaminopyridine, 2-amino-4-[(2-hydroxyethyl)-amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethylbenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)-benzene, 2,4-diamino-1,5-di(2-hydroxyethoxy)-benzene, 1-(2-(aminoethoxy)-2,4-diaminobenzene, 2-amino-1-(2-hydroxyethoxy)-4-methylaminobenzene, 2,4-diaminophenoxy acetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]-aniline, 3-[(2-aminoethyl)amino]aniline, 1,3-di-(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]-acetamide, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1-7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

7. Agent according to one of Claims 2 to 6, **characterized in that** the developer substances and coupler substances, based on the total amount of the colouring agent, are in each case present in a total amount of from 0.005 to 20% by weight.

8. Agent according to one of Claims 2 to 7, **characterized in that** it additionally comprises at least one direct dye.

9. Agent according to one of Claims 2 to 8, **characterized in that** it is a hair colouring agent.

## Revendications

1. Dérivés de N-benzyl-p-phénylènediamine de formule générale (I) ou leurs sels solubles dans l'eau, physiologiquement acceptables, 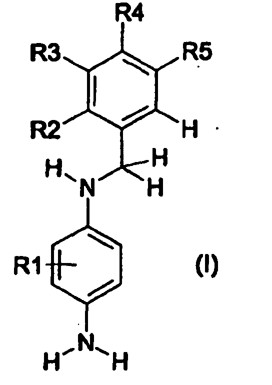 dans laquelle
R1 représente hydrogène, un groupe alkyle en C₁ à C₄ ou un groupe hydroxyalkyle en C₁ à C₄ ;
R2 représente hydrogène, un atome d'halogène (F, Cl, Br, I), un groupe cyano, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe nitro, un groupe (alkyle en C₁ à C₄)amino, un groupe di (alkyle en C₁ à C₄)amino, un groupe di(hydroxyalkyle en C₁ à C₄)amino, un groupe (hydroxyalkyle en C₁ à C₄)amino, un groupe trifluorométhane, un groupe -C(O)CH₃, un groupe -C(O)CF₃, un groupe -Si(CH₃)₃, un groupe hydroxyalkyle en C₁ à C₄, un groupe dihydroxyalkyle en C₃ à C₄ ou un groupe morpholino ;
R3 représente hydrogène, un atome d'halogène, un groupe hydroxy, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe (alkyle en C₁ à C₆)amino, un groupe di (alkyle en C₁ à C₆)amino, un groupe di (hydroxyalkyle en C₁ à C₄)amino, un groupe hydroxy(alkyle en C₁ à C₄)amino, un groupe trifluorométhane, un groupe acétamido, un groupe -C(O)CH₃, un groupe -C(O)CF₃, un groupe -Si(CH₃)₃, un groupe hydroxyalkyle en C₁ à C₄ ou un groupe dihydroxyalkyle en C₃ à C₄ ;
R4 représente un atome d'halogène, un groupe hydroxy, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe (alkyle en C₁ à C₆)amino, un groupe di (alkyle en C₁ à C₆)amino, un groupe di(hydroxyalkyle en C₁ à C₄)amino, un groupe hydroxy(alkyle en C₁ à C₄)amino, un groupe trifluorométhane, un groupe acétamido, un groupe -C(O)CH₃, un groupe -C(O)CF₃, un groupe -Si(CH₃)₃, un groupe hydroxyalkyle en C₁ à C₄ ou un groupe dihydroxyalkyle en C₃ à C₄,
ou R3 et R4 forment ensemble un pont -O-CH₂-O- ;
R5 représente hydrogène, un groupe hydroxy ou un groupe alkyle en C₁ à C₆, à condition que
(i) R4 ne représente pas un groupe méthyle, un groupe hydroxy, un groupe diméthylamino, un atome de brome, ni un atome de chlore lorsque R1=R2=R3=R5= hydrogène, et
(ii) au moins un des radicaux résiduels R1 à R5 soit différent de hydrogène lorsque R3 et R4 ou R4 et R5 représentent un groupe méthoxy, et
(iii) R4 ne représente pas un groupe hydroxy lorsque R1 et R2 représentent un atome d'hydrogène et R3 et R5 représentent un groupe t-butyle.

2. Agent pour la coloration par oxydation de fibres kératiniques à base d'une combinaison substance de développement-substance de couplage, **caractérisé en ce qu'**il contient comme substance de développement au moins un dérivé de N-benzyl-p-phénylènediamine de formule générale (I) ou ses sels solubles dans l'eau, physiologiquement acceptables, 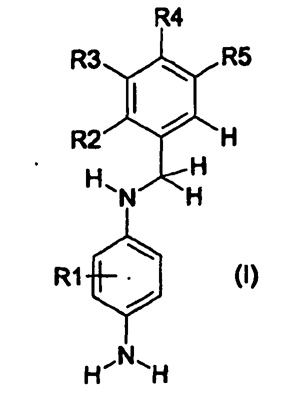 dans laquelle
R1 représente hydrogène, un groupe alkyle en C₁ à C₄ ou un groupe hydroxyalkyle en C₁ à C₄ ;
R2 représente hydrogène, un atome d'halogène (F, Cl, Br, I), un groupe cyano, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe nitro, un groupe (alkyle en C₁ à C₄)amino, un groupe di (alkyle en C₁ à C₄)amino, un groupe di(hydroxyalkyle en C₁ à C₄)amino, un groupe (hydroxyalkyle en C₁ à C₄)amino, un groupe trifluorométhane, un groupe -C(O)CH₃, un groupe -C(O)CF₃, un groupe -Si(CH₃)₃, un groupe hydroxyalkyle en C₁ à C₄, un groupe dihydroxyalkyle en C₃ à C₄ ou un groupe morpholino ;
R3 représente hydrogène, un atome d'halogène, un groupe hydroxy, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe (alkyle en C₁ à C₆)amino, un groupe di(alkyle en C₁ à C₆)amino, un groupe di(hydroxyalkyle en C₁ à C₄)amino, un groupe hydroxy(alkyle en C₁ à C₄)amino, un groupe trifluorométhane, un groupe acétamido, un groupe -C(O)CH₃, un groupe -C(O)CF₃, un groupe -Si(CH₃)₃, un groupe hydroxyalkyle en C₁ à C₄ ou un groupe dihydroxyalkyle en C₃ à C₄ ;
R4 représente un atome d'halogène, un groupe hydroxy, un groupe alcoxy en C₁ à C₄, un groupe hydroxyalcoxy en C₁ à C₄, un groupe alkyle en C₁ à C₆, un groupe (alkyle en C₁ à C₄)thioéther, un groupe mercapto, un groupe (alkyle en C₁ à C₆)amino, un groupe di (alkyle en C₁ à C₆)amino, un groupe di(hydroxyalkyle en C₁ à C₄)amino, un groupe hydroxy(alkyle en C₁ à C₄)amino, un groupe trifluorométhane, un groupe acétamido, un groupe -C(O)CH₃, un groupe -C(O)CF₃, un groupe -Si(CH₃)₃, un groupe hydroxyalkyle en C₁ à C₄ ou un groupe dihydroxyalkyle en C₃ à C₄,
ou R3 et R4 forment ensemble un pont -O-CH₂-O- ;
R5 représente hydrogène, un groupe hydroxy ou un groupe alkyle en C₁ à C₆.

3. Agent selon la revendication 2, **caractérisé en ce que** le composé de formule (I) est choisi dans le groupe constitué par le N-((4-hydroxyphényl)méthyl)-1,4-diaminobenzène ; le N-((4-hydroxy-3,5-diméthylphényl)méthyl)-1,4-diaminobenzéne; le N-((4-(2-hydroxyéthoxy)phényl)méthyl)-1,4-diaminobenzène; le N-benzo-[1,3]-dioxol-5-ylméthyl-1,4-diaminobenzène; le N-(4-[(4-aminophénylamino)méthyl]phényl}acétamide et le N-((4-méthoxyphényl)méthyl)-1,4-diaminobenzène ainsi que les sels physiologiquement acceptables de ces composés.

4. Agent selon la revendication 2 ou 3,
**caractérisé en ce que** le dérivé de N-benzyl-p-phénylènediamine de formule (I) est contenu en une quantité de 0,005 à 20% en poids.

5. Agent selon l'une quelconque des revendications 2 à 4, **caractérisé en ce qu'**il présente un pH de 6,5 à 11,5.

6. Agent selon l'une quelconque des revendications 2 à 5, **caractérisé en ce que** la substance de couplage est choisie dans le groupe constitué par la 2,6-diaminopyridine, le 2-amino-4-[(2-hydroxyéthyl)amino]-anisol, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]-aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)-amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

7. Agent selon l'une quelconque des revendications 2 à 6, **caractérisé en ce que** les substances de développement et les substances de couplage, par rapport à la quantité totale de colorant, sont contenues à chaque fois en une quantité totale de 0,005 à 20% en poids.

8. Agent selon l'une quelconque des revendications 2 à 7, **caractérisé en ce qu'**il contient en outre au moins un colorant montant directement sur la fibre.

9. Agent selon l'une quelconque des revendications 2 à 8, **caractérisé en ce qu'**il s'agit d'un agent de teinture pour cheveux.
